# EUROPÄISCHE PATENTSCHRIFT

(11) **EP 2 983 622 B1**
(45) Veröffentlichungstag und Bekanntmachung des Hinweises auf die Patenterteilung: **07.12.2016**
(21) Anmeldenummer: 14736616.5
(22) Anmeldetag: 25.06.2014
(51) Int. Cl.: A61F 2/44, A61F 2/46, A61F 2/30

(54) **ZWISCHENWIRBEL-KÖRBCHEN**
INTERBODY CAGE
CAGE INTERSOMATIQUE

(30) Priorität: 14.08.2013 DE 202013007341 U; 25.04.2014 DE 202014003441 U
(43) Veröffentlichungstag der Anmeldung: 17.02.2016
(73) Patentinhaber: Joimax GmbH, 76227 Karlsruhe (DE)
(72) Erfinder: RIES, Wolfgang, 76351 Linkenheim (DE)
(74) Vertreter: Lichti - Patentanwälte Partnerschaft mbB
(86) Internationale Anmeldenummer: PCT/EP2014/001719
(87) Internationale Veröffentlichungsnummer: WO 2015/022039

(56) Entgegenhaltungen:
- WO-A1-2013/063194
- FR-A1- 2 977 474
- TW-A- 201 240 653
- US-A1- 2005 112 397
- US-A1- 2006 074 488
- US-A1- 2010 152 853
- US-A1- 2011 014 081
- US-A1- 2013 096 685

## Beschreibung

Die Erfindung betrifft ein Zwischenwirbel-Körbchen.

Zwischenwirbel-Körbchen werden im Englischen als Interbody Cages bezeichnet. Bei einer Reihe von Wirbelsäulenschäden, insbesondere Bandscheibenschäden, wie Wirbelgleiten und Instabilität nach einem Bandscheibenvorfall; durch Stenose und Degeneration wird eine Versteifung der Wirbelsäule zwischen den beiden von dem Schaden betroffenen Wirbelkörpern durchgeführt. Hierzu werden Körbchen nach entfernen einer beschädigten und/oder (nerven-)schädigenden Bandscheibe aus dem Zwischenwirbelraum oder Bandscheibenfach zwischen zwei unmittelbar übereinander befindlichen Wirbeln der Wirbelsäule eingesetzt, um diese auf dem vorgegebenen Abstand zu halten. Es erfolgt eine Versteifung der Wirbelsäule zumindest im Bereich dieser beiden Wirbel. Dies wird als z.B. (lumbale) Wirbelfusion (Lumbar Interbody Fusion - LIF) bezeichnet. Gegebenenfalls werden die Wirbel noch - in minimal-invasiver Spinalchirurgie - mit einer Stab-Schrauben-Einheit, Facettengelenksschrauben oder translaminären Schrauben versehen und gegen das zwischen ihnen liegende Körbchen verspannt. Insofern ist es auch wünschenswert, dass die Knochen mit dem Körbchen verwachsen. Ein solches wird daher in der Regel mit einer rauen Oberfläche versehen.

Der Erfindung liegt die Aufgabe zugrunde, ein Zwischenwirbel-Körbchen zu schaffen, das verbesserte Eigenschaften aufweist.

In bevorzugter Ausgestaltung ist dabei vorgesehen, dass ein die äußere Kontur bestimmender Rahmen und innerhalb desselben befindliche Netz- oder Gitterbereich einstückig ausgebildet sind, wobei insbesondere durch Sintern, wie mittels Elektronenstrahlschmelzen oder Lasersintern, hergestellt ist.

Erfindungsgemäß wird die genannte Aufgabe mit einem Körbchen gelöst, das zumindest Bereiche mit netz- oder gitterförmiger Struktur aufweist. Es ist demgemäß mit netz- oder gitterförmigen Bereichen ausgebildet.

Die Herstellung eines solchen Körbchens geschieht vorzugsweise durch Sintern, insbesondere mittels (selektiven) Elektronenstrahlschmelzen ((Selective) Electron Beam Melting, (S)EBM) oder mittels Lasersintern. (LST). Demgemäß ist das Körbchen durch Sintern wie mittels Elektronenstrahlschmelzen oder Lasersintern, hergestellt.

Die netz- oder gitterförmigen Bereiche reichen vorzugsweise von einer Ober- oder Seitenfläche bis zur parallelen gegenüberliegenden Unter- bzw. Seitenfläche. Hierdurch wird erreicht, dass Knochengewebe nicht nur äußerlich oberflächlich an den Körbchen anwächst, sondern dieses in die Hohlräume der gitter- oder netzartigen Struktur einwächst und das Körbchen so vollständig durchdringen kann bzw. das Knochenwachstum gefördert wird. Hierdurch wird eine feste Verbindung zwischen Körbchen und benachbartem Wirbelkörper erreicht.

Die Körbchen können bevorzugt eine Länge von 22 mm bis 40 mm, und eine Breite in der Größenordnung zwischen 10 mm und 15 mm und jeweils eine unterschiedliche Höhe zwischen 6 mm bis 16 mm je nach Konstitution des Patienten und dem Ort der Einbringung bzw. den Wirbelkörpern und deren Bandscheibenfach, in die das Körbchen eingebracht werden soll, aufweisen. Mit diesen Breiten und Höhen sind die Körbchen durch eine Arbeitshülse in das Bandscheibenfach zwischen den Wirbelkörpern einbringbar.

Des Weiteren ist das Körbchen mit einem äußeren Rahmen, der aus massiven Tragteilen besteht, und mit einem inneren Gitterkörper versehen. Durch die Ausbildung des Körbchens mit zwei Strukturelementen, nämlich einem - äußeren - Rahmen aus kompaktem Material und in kompakter Struktur und einem inneren Gitterkörper mit der genannten netz- oder gitterartigen Struktur wird bei Beibehaltung der vorgenannten Vorteile des vollständigen Durchwachsens von Knochenmaterial durch das Körbchen bzw. dessen gitterartige Struktur dem Körbchen eine hinreichende Festigkeit und Steifigkeit verliehen.

Zudem ist der Gitterkörper nur an parallel in einer Richtung, nämlich Querrichtung verlaufenden Flächen mit dem Rahmen verbunden, aber in zu diesen Flächen unter einem endlichen Winkel verlaufenden Flächen und Kanten nicht mit dem Rahmen in Verbindung steht. Hierdurch wird in gewisser Weise eine Entkopplung des äußeren Rahmens und des Gitterkörpers des Körbchens erreicht, indem beide in der Längsoder Haupterstreckungsrichtung des Körbchens (das Körbchen ist in Einführrichtung länger als in Querrichtung und in seiner Höhe) beide entkoppelt sind. Wenn der Rahmen beispielsweise im Bereich von ihm bildenden Rippen unter auf ihn ausgeübten Druck nachgibt, so wird dieser nicht auf den Gitterkörper übertragen. Letzterer bleibt damit unbeeinträchtigt und auch in ihn eingewachsene Knochenstruktur wird nicht beeinträchtigt oder beschädigt.

Dabei ist in bevorzugter Weiterbildung vorgesehen, dass obere und untere Flächen des Gitterkörpers gleiche Abmessungen aufweisen, wie von Rahmenkomponenten umgebende Freiräume, die die genannten Flächen des Gitterkörpers umgeben.

In weiterer Ausbildung des erfindungsgemäßen Körbchens ist vorgesehen, dass der Rahmen einen Hohlraum umgibt, in dem der Gitterkörper angeordnet ist. Eine Weiterbildung sieht vor, dass der Rahmen in seiner Längsrichtung verlaufende Längsrippen aufweist. Eine äußerst bevorzugte Ausgestaltung sieht vor, dass benachbarte Längsrippen mittig durch Querrippen verbunden sind. Hierdurch wird auch bei längeren Körbchen die Stabilität des Rahmens und damit des Körbchens selbst erhöht. Die Netz- oder Gitterstruktur des Gitterkörpers des Körbchens kann in verschiedener Weise ausgestaltet sein. In äußerst bevorzugter Ausgestaltung ist vorgesehen, dass der Gitterbereich oder Gitterkörper eine Diamantstruktur aufweist.

In weiterer Ausbildung des Körbchens kann vorgesehen sein, dass es einen durchgehenden Durchlass aufweist. Der Durchbruch erstreckt sich dabei in Längsrichtung durch den Rahmen und insbesondere auch durch den Gitterkörper selbst. Hierdurch wird erreicht, dass das Körbchen - mittels eines Einsetzinstruments - über einen bis in den Zwischenwirbelraum oder das Bandscheibenfach liegenden Führungsdraht - durch eine rohrförmige Schleuse oder ein Endoskoprohr - eingeführt werden kann.

Weitere bevorzugte Ausgestaltungen des erfindungsgemäßen Körbchens zeichnen sich dadurch aus, dass die gitterförmigen Bereiche oder der Gitterkörper Gitter-Öffnungsdurchmesser jeder Öffnung von 0,5 mm bis 3,5 mm aufweisen, dabei vorzugsweise an der Außenseite des Körbchens Öffnungsdurchmesser jeder Öffnung in der Größenordnung von 0,5 mm bis 0,7 mm aufweisen und/oder die gitterförmigen Bereiche oder der Gitterkörper im Inneren des Körbchens Öffnungen oder Durchbrüche mit Durchmessern von 0,5 mm bis 3,5 mm aufweisen, insbesondere durch einen proximalen Verbindungsbereich zur Verbindung des Körbchens mit einem Einsetzinstrument.

Ein Körbchen kann - wie gesagt - insbesondere durch (selektives) Elektronenstrahlschmelzen ((Selective) Electron Beam Melting (S)EBM) oder Lasersinterungstechnik (LST) aus Titanlegierung, insbesondere Ti6Al4V gemäß ISO 5832-3 hergestellt werden. Das Bauteil - Körbchen - wird durch Aufschmelzen von Metallpulver mittels eines Elektronenstrahls oder Laserstrahls im Hochvakuum her-gestellt. Hierdurch sind Hinterschneidungen ohne verlorene Formen oder Kerne erzeugbar. Durch einen Elektronenstrahl oder Laserstrahl als Energiequelle wird dabei das Metall-pulver gezielt aufgeschmolzen, wodurch kompakte Bauteile nahezu beliebiger Geometrie direkt aus Konstruktionsdaten hergestellt werden können. ES wird abwechselnd eine Pulver-lage mittels eines Rakels auf die vorherige aufgebracht und mittels des Elektronenstrahls belichtet. Auf diese Weise wird das gewünschte Bauteil schichtweise generiert.

Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnung im Einzelnen erläutert sind. Dabei zeigt:

In bevorzugter Ausgestaltung ist vorgesehen, dass eine proximale Stirnseite eine Zähnung aufweist. Diese kann auch in zwei- oder mehrfacher Ausführung vorhanden sein. Die weitere Ausgestaltung der Zähnung kann auf das Einsetzinstrument abgestimmt sein. Hierdurch werden verschiedene Winkelausrichtungen zwischen Körbchen und Einsetzinstrument und eine optimale Positionierbarkeit des Körbchens ermöglicht.
- Fig. 1: eine Seitenansicht zweier Wirbel mit zwischen diesen eingesetzten Zwischenwirbel-Körbchen;
- Fig. 2: eine perspektivische Draufsicht auf einen Wirbel mit aufsitzendem Körbchen;
- Fig. 3: eine erste Ausführungsform eines erfindungsgemäßen Körbchens in perspektivischer Darstellung;
- Fig. 4: das Körbchen der Fig. 3 im Längsschnitt;
- Fig. 5: das Rahmenteil des Körbchens der Fig. 3, 4 in perspektivischer Darstellung;
- Fig. 6: der Gitterkörper des Körbchens der Fig. 3, 4 in perspektivischer Darstellung;
- Fig. 7: einen Längsschnitt durch eine andere Ausführungsform des erfindungsgemäßen Körbchens;
- Fig. 8: eine perspektivische Darstellung des äußeren Rahmens des erfindungsgemäßen Körbchens der Fig. 7;
- Fig. 9: eine Draufsicht auf das erfindungsgemäße Körbchen der Fig. 7, 8;
- Fig. 10: eine perspektivische Darstellung des Gitterkörpers des Körbchens der Fig. 7 - 9;
- Fig. 11: ein erfindungsgemäßes Körbchen mit einem Einsetzinstrument; und
- Fig. 12: eine vergrößerte Darstellung des Körbchens am distalen Bereich des Einsetzinstruments der Fig. 11.

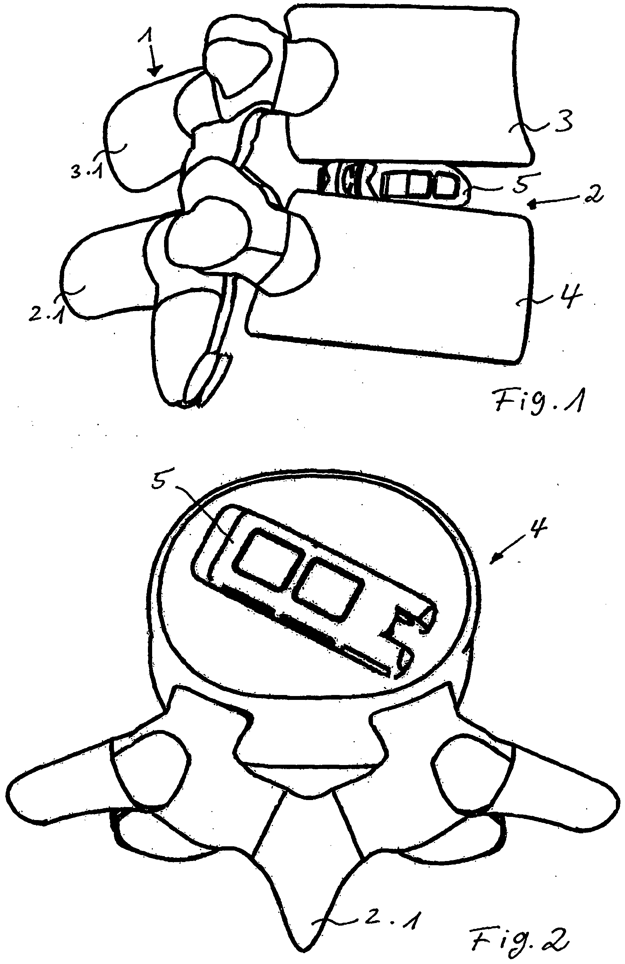

Wenn aufgrund von Schäden einer Bandscheibe an der Wirbelsäule eines Patienten die Bandscheibe aus dem Zwischenwirbelraum 2 zwischen zwei übereinanderliegenden Wirbeln 3, 4 einer Wirbelsäule 1 zu entfernen ist, wird stattdessen ein Körbchen (Cage) eingesetzt, um die Wirbel auf geeignetem Abstand zu halten, wie dies in den Fig. 1 und 2 gezeigt ist. Hiermit ist in der Regel eine Versteifung der Wirbelsäule in diesem Bereich verbunden, so dass die beiden Wirbel 3, 4, zwischen denen das Körbchen 5 eingesetzt ist, nicht mehr relativ zueinander beweglich sind. Eine Verspannung kann zusätzlich durch ein Stab-Schrauben-System, Facettengelenksschrauben oder translaminären Schrauben erfolgen. Weiterhin wird angestrebt, dass das Körbchen 5 mit den Wirbeln verwächst und ist demgemäß ausgebildet.

Das Körbchen 5 kann unter unterschiedlichen Richtungen zu den Dornfortsätzen 2.1, 3.1 eingebracht werden, im dargestellten Ausführungsbeispiel als Oblique-Cage (O-Cage) mit einer Winkelausrichtung von etwa 6° zu den Dornfortsätzen. Posterior-Cages (P-Cages) sind kürzer als O-Cages und weisen je nach Patient eine Länge von 24 mm bis 30 mm auf. Sie werden dorsal neben dem Dornfortsatz leicht schräg zu diesem in das Bandscheibenfach eingeführt.

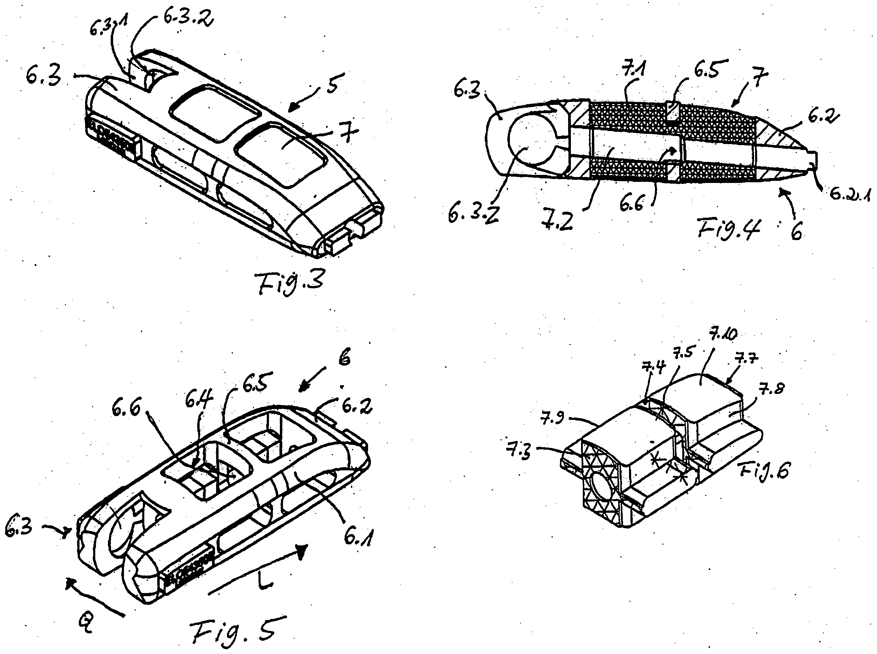

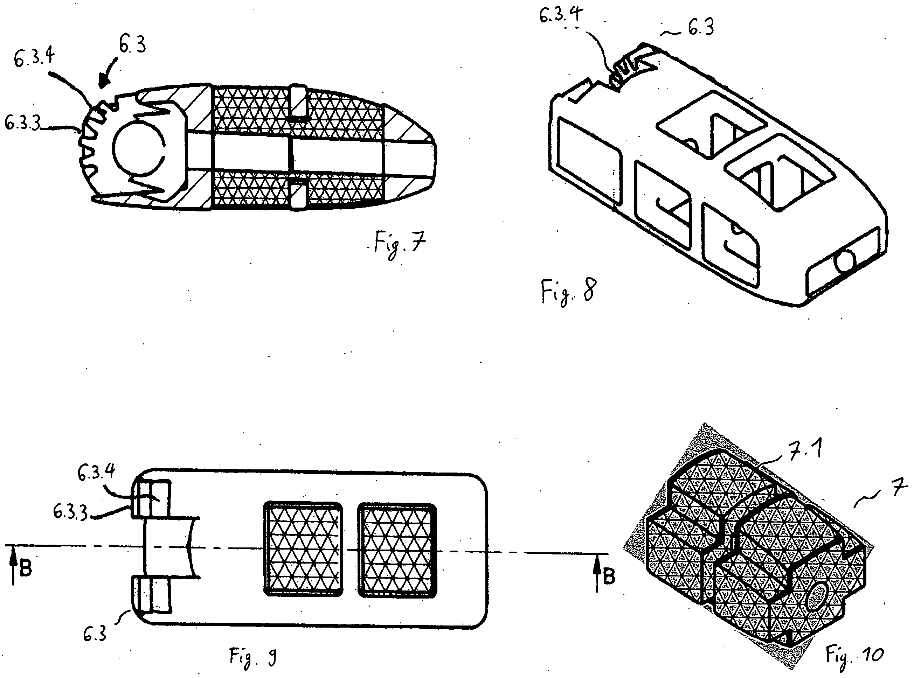

Die Höhe des Körbchens 5 muss der - ursprünglichen - Höhe des Zwischenwirbelraums bzw. Bandscheibenfachs 2 angepasst sein, die von Mensch zu Mensch unterschiedlich sein kann. Es sind daher je nach Konstitution des Patienten Körbchen 5 mit unterschiedlicher Höhe vorzusehen. In der Fig. 6 ist ein Körbchen mit einer Höhe von 8 mm, in den Fig. 7 bis 8 ein Körbchen mit einer Höhe von 14 mm dargestellt. Üblicherweise wird man Körbchen in mm-Schritten vorsehen.

Ein erfindungsgemäßes Körbchen besteht in den Figuren der dargestellten bevorzugten Ausführungsform grundsätzlich aus zwei Hauptbestandteilen, nämlich aus einem - äußeren - massiven Rahmen und einem - inneren - Gitterkörper 7.

Der Gitterkörper 7 weist eine Gitterstruktur, vorzugsweise eine Diamantgitterstruktur mit dünnen Rippen 7.1 und zwischen diesen freien Zwischenräumen auf, wobei die Abmessungen der Rippen 7.1, insbesondere deren Stärke (in senkrechter Richtung zur Erstreckungsrichtung der Rippen zwischen zwei Knotenpunkten, an denen sie jeweils mit weiteren Rippen des Gitters verbunden sind) gering ist gegenüber sämtlichen Abmessungen von Strukturkomponenten des Rahmens 6, wie beispielsweise eine Breite von Rippen des Rahmens 6. Die Größenverhältnisse betragen mindestens 1 zu 10. Entsprechendes gilt für die Länge von Streben 7.1 des Gitterkörpers 7 zwischen zwei Knotenpunkten und Längsabmessungen von Strukturteilen des Rahmens 6, wie den genannten Rippen, so dass auch hier das Verhältnis mindestens 1 zu 10 beträgt.

Auch wenn das Körbchen 5 aus zwei Hauptbestandteilen - Rahmen 6 und Gitterkörper 7 - besteht, so ist es doch einstückig.

Der Rahmen 6 weist vier Längsrippen 6.1 auf, die einen distalen Endbereich 6.2 des Körbchens 5 und einen proximalen Verbindungsbereich 6.3 verbinden und mit diesem einen Hohlraum 6.4 einschließen, in dem sich beim fertigen Körbchen der Gitterkörper befindet. Die Rippen 6.1 sind mittig, d.h. etwa auf der Hälfte ihrer Länge, durch Querrippen 6.5 miteinander verbunden und zwar jeweils benachbarte Rippen 6.1. Demgemäß verbleibt zwischen den Querrippen 6.5 ein Durchlass 6.6. Entsprechende Durchlässe 6.2.1 und 6.3.1 finden sich auch im distalen Endbereich 6.2 und im proximalen Verbindungsbereich 6.3. Darüber hinaus ist auch der Gitterkörper 7 mit einem Längsdurchlass 7.2 versehen, der mit den vorgenannten Durchlässen fluchtet.

In äußerst bevorzugter Ausgestaltung sind Gitterkörper 7 und Rahmen 6 nur in senkrecht zur Längsrichtung L und damit an quer verlaufenden (Flächen-)Bereichen miteinander - einstückig - verbunden und zwar nur mit den Flächen 7.3, 7.4, 7.5, 7.7 (Fig. 6 und 10).

Demgegenüber sind Längsflächen, wie 7.8 und auch Längskanten, wie 7.9 des Gitterkörpers 7 nicht fest mit dem Rahmen 6 verbunden. Darüber hinaus entsprechen insbesondere die Abmessungen der oberen Flächen 7.10 und der diesen parallel auf der Unterseite der Gitterkörper 7 gegenüberliegenden unteren Flächen dem den Ausnehmungen bzw. den durch die Längsrippen 6.1, den Querrippen 6.5 und den Stirnbereichen 6.2 sowie den proximalen Verbindungsbereich 6.3 freigelassenen Bereichen. Dies führt dazu, dass bei Einwirkung von Druckbelastung auf die Längsrippen 6.1 durch die Wirbelkörper 2, 3 diese nicht an den Längsseiten auf den Gitterkörper 7 übertragen wird und dieser damit unverformt bleibt und seine Aufgabe, ein Einwachsen von Knochenmaterial in diese Waben- oder Zwischenräume des Gitterkörpers 6 zu gewährleisten, auch unter diesen Umständen erfüllen kann. Der durchgehende Längsdurchlass des Körbchens 5 ermöglicht, dass dieser über einen liegenden Führungsdraht in das Wirbelzwischenfach eingeführt werden kann.

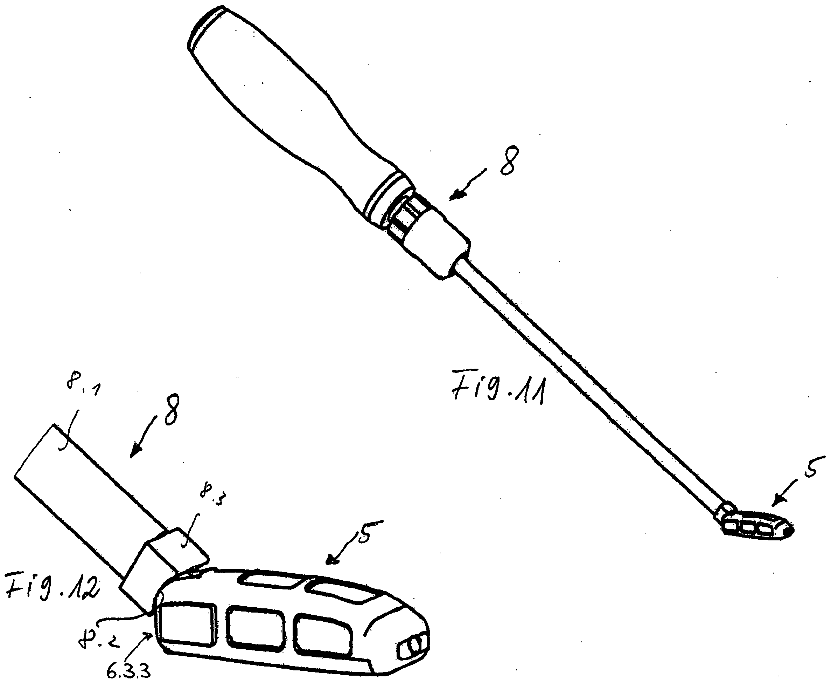

Bei der Ausgestaltung der Fig. 7 bis 10 eines erfindungsgemäßen Körbchens 5 weist der proximale Verbindungsbereich 6.3 desselben (am Rahmen 6) an seiner proximalen gerichteten äußeren Stirnseite 6.3.3 Zähnungen 6.3.4 auf. Diese dienen dazu, bei Festlegen des Körbchens 5 an dem distalen Ende eines Einsetzinstruments 8 (Fig. 12) durch axiales Verspannen zwischen einem hammerartigen Verriegelungsglied des Einsetzinstruments und einem Widerlager 8.3 desselben eine vorgegebene angulare Ausrichtung zwischen Einsetzinstrument 8 und Körbchen 5 zu sichern. Die Zähnungen 6.3.4 sind von den nach unten auf einen Kreisbogen aufeinanderfolgende Zähne gebildet. Es findet sich jeweils eine Zähnung 6.3.4 auf jeder Seite des proximalen Durchlass 6.2.1 des Körbchens.

Die Ausgestaltung (z.B. Anzahl, Abstand, Form) der Zähnungen 6.3.4 können auf das Einsetzinstrument 8 und/oder dem Eingriff abgestimmt sein. Dadurch ist zum Einen eine optimale Kompatibilität zum Einsetzinstrument 8, eine erhöhte Stabilität der Verbindung zwischen Widerlager 8.3 des Einsetzinstrument 8 und proximalem Verbindungsbereich 6.3 des Körbchens 5 und zum Anderen eine Vielzahl von Verbindungswinkeln ermöglicht.

Das Einbringen eines erfindungsgemäßen Körbchens 5 geschieht mittels eines Einsetzinstruments 8, wie dies in den Fig. 11 und 12 dargestellt ist. Ein sich durch ein äußeres Rohr 8.1 entstandenes Verriegelungselement weist ein um seine Längsachse verdrehbares hammerartige Verriegelungsteil (beide nicht dargestellt) auf, das beispielsweise bei der Darstellung der Fig. 3 bis 5 des Körbchens 6 in senkrechter Ausrichtung in den Durchlass 6.3.1 des proximalen Verbindungsbereichs 6.3 des Körbchens 5 eingeführt wird. Hierzu weist das Körbchen 5 an seiner proximalen Stirnseite eine hinterschnittene Öffnung auf, deren Öffnungsquerschnitt dem Verriegelungselement des Einsetzinstruments 8 entspricht; die hinterschnittene Öffnung bildet ein Verriegelungselement am Körbchen 5, wodurch eine Verriegelung von Einsetzinstrument 8 und Körbchen 5 ermöglicht wird.

Das hammerartige Verriegelungsteil des Einsetzinstruments wird im Folgenden relativ zu dem äußeren Rohr 8.1, um 90° geschwenkt, so dass es in Hinterschneidungen 6.3.2 auf der Innenseite der Wandung des proximalen Verbindungsbereichs 6.3 zum Greifen kommt. Mittels Spannungseinrichtungen am proximalen Ende des Einsetzinstruments 8 werden das hammerartige Verriegelungsteil und konkav gebogene Stirnkanten 8.2 an einem distal am äußeren Rohr 8.1 vorgesehenen Widerlager 8.3 unter Zwischenlage von Verriegelungsteil hintergriffenen proximalen Verbindungsbereich 6.3 des Körbchens 5 gegeneinander verspannt. Hierdurch wird das Körbchen 5 fest am Einsetzinstrument gehalten. Damit ein Bewegen des Körbchens 5 in seiner Erstreckungsrichtung und mit einer Komponente zur Erstreckungsrichtung des Einsetzinstruments 8 ermöglicht. Soweit der proximale Verbindungsbereich 6.3 des Körbchens 5 an seiner (äußeren) Stirnseite 6.3.3 eine Zähnung 6.3.4 aufweist, ist hierdurch auch eine eingenommene Winkelstellung zwischen Einsetzinstrument 8 und Körbchen 5 gesichert. Dennoch ist, wie insbesondere die Darstellung der Ausbildung der Ausnehmung 6.3.2 mit Kreisform gemäß Fig. 4 zeigt, ein Verschwenken zwischen Einsetzinstrument 8 und Körbchen 5 in senkrechter Richtung zur Längserstreckung L von beiden um einen beträchtlichen Winkel von bis zu 30° und mehr möglich.

## Patentansprüche

1. Zwischenwirbel-Körbchen mit einem äußeren Rahmen (6) aus massiven Tragteilen (6.1, 6.2, 6.3, 6.5) und mit einem inneren Gitterkörper, **dadurch gekennzeichnet, dass** der Gitterkörper (7) nur an parallel in einer Richtung, nämlich Querrichtung, verlaufenden Flächen (7.3, 7.4, 7.5, 7.6) mit dem Rahmen (6) verbunden ist, aber in zu diesen Flächen (7.3, 7.4, 7.5, 7.6) unter einem endlichen Winkel verlaufenden Flächen (7.8) und Kanten (7.9) nicht mit dem Rahmen (6) in Verbindung steht.

2. Körbchen nach Anspruch 1, **dadurch gekennzeichnet, dass** ein die äußere Kontur bestimmender Rahmen und innerhalb desselben befindliche Netz- oder Gitterbereiche einstückig ausgebildet sind.

3. Körbchen nach einem der vorangehenden Ansprüche, **dadurch gekennzeichnet, dass** es durch Sintern, wie mittels Lasersintern, hergestellt ist.

4. Körbchen nach einem der Ansprüche 1 bis 3, **dadurch gekennzeichnet, dass** es mittels Elektronenstrahlschmelzen, insbesondere selektivem Elektronenstrahlschmelzen, hergestellt ist.

5. Körbchen nach einem der Ansprüche 1 bis 4, **dadurch gekennzeichnet, dass** obere und untere Flächen (7.10) des Gitterkörpers (7) gleiche Abmessungen aufweisen wie von Rahmenkomponenten (6.1, 6.2, 6.3, 6.5) umgebende Freiräume, die die genannten Flächen (7.10) des Gitterkörpers (7) umgeben.

6. Körbchen nach einem der Ansprüche 1 bis 5, **dadurch gekennzeichnet, dass** der Rahmen (6) einen Hohlraum umgibt, in dem der Gitterkörper (7) angeordnet ist.

7. Körbchen nach einem der Ansprüche 1 bis 6, **dadurch gekennzeichnet, dass** der Rahmen (4) in seiner Längsrichtung verlaufende Längsrippen (6.1) aufweist, wobei insbesondere benachbarte Längsrippen (6.1) mittig durch Querrippen (6.5) verbunden sind.

8. Körbchen nach einem der vorangehenden Ansprüche, **dadurch gekennzeichnet, dass** der Gitterbereich oder Gitterkörper (6) eine Diamantstruktur aufweist.

9. Körbchen nach einem der vorangehenden Ansprüche, **dadurch gekennzeichnet, dass** es einen durchgehenden Durchlass (7.2, 6.6) aufweist.

10. Körbchen nach einem der vorangehenden Ansprüche, **dadurch gekennzeichnet, dass** die gitterförmigen Bereiche oder der Gitterkörper (7) Gitter-Öffnungsdurchmesser jeder Öffnung von 0,5 mm bis 3,2 mm aufweisen, dabei vorzugsweise an der Außenseite des Körbchens Öffnungsdurchmesser jeder Öffnung in der Größenordnung von 0,5 mm bis 0,7 mm aufweisen.

11. Körbchen nach einem der vorangehenden Ansprüche, **dadurch gekennzeichnet, dass** die gitterförmigen Bereiche oder der Gitterkörper (7) im Inneren des Körbchens Öffnungen oder Durchbrüche mit Durchmessern von 0,5 mm bis 3,2 mm aufweisen.

12. Körbchen nach einem der vorangehenden Ansprüche, **gekennzeichnet durch** einen proximalen Verbindungs-bereich (6.3) zur Verbindung des Körbchens mit einem Einsetzinstrument (8), wobei insbesondere der Verbindungsbereich (6.3) auf der Innenseite von Seitenwänden des Körbchens (5) Vertiefungen (6.3.2) aufweist, die eine angular bewegliche, hinter-schneidende Verbindung mit dem Einsetzinstrument (8) ermöglichen.

13. Körbchen nach einem der vorangehenden Ansprüche, **dadurch gekennzeichnet, dass** ein Verbindungsbereich (6.3) in der Oberseite des Körbchens (5) einen Ausschnitt aufweist, der eine angulare Beweglichkeit zwischen Körbchen (5) und Einsetzinstrument (8) ermöglicht.

14. Körbchen nach einem der vorangehenden Ansprüche, **dadurch gekennzeichnet, dass** eine proximale Stirnseite (6.3.3) eine Zähnungen (6.3.4) aufweist, wobei insbesondere die Zähnungen (6.3.4) durch in vertikaler Richtung auf Kreisabschnitten aufeinanderfolgend hohl gebildet sind sowie die Zähnungen (6.3.4) beidseitig eines an der proximalen Stirnseite mittig ausgebildeten proxialen Durchlasses (6.3.1) angeordnet sind.

## Claims

1. Intervertebral cage with an outer frame (6) composed of solid support parts (6.1, 6.2, 6.3, 6.5) and with an inner grid body, **characterized in that** the grid body (7) is connected to the frame (6) only at surfaces (7.3, 7.4, 7.5, 7.6) extending in parallel in one direction, namely the transverse direction, but they are not connected to the frame (6) at surfaces (7.8) and edges (7.9) extending at a finite angle in relation to these surfaces (7.3, 7.4, 7.5, 7.6).

2. Cage according to Clam 1, **characterized in that** a frame defining the outer contour and lattice or grid areas located within said frame are formed in one piece.

3. Cage according to either of the preceding claims, **characterized in that** it is produced by sintering, for example by means of laser sintering.

4. Cage according to one of Claims 1 to 3, **characterized in that** it is produced by means of electron beam melting, in particular by means of selective electron beam melting.

5. Cage according to one of Claims 1 to 4, **characterized in that** upper and lower surfaces (7.10) of the grid body (7) have the same dimensions as free spaces which are surrounded by frame components (6.1, 6.2, 6.3, 6.5) and which surround said surfaces (7.10) of the grid body (7).

6. Cage according to one of Claims 1 to 5, **characterized in that** the frame (6) surrounds a cavity, in which the grid body (7) is arranged.

7. Cage according to one of Claims 1 to 6, **characterized in that** the frame (4) has longitudinal ribs (6.1) extending in its longitudinal direction, wherein adjacent longitudinal ribs (6.1) in particular are connected centrally by transverse ribs (6. 5).

8. Cage according to one of the preceding claims, **characterized in that** the grid area or grid body (6) has a diamond structure.

9. Cage according to one of the preceding claims, **characterized in that** it has a continuous passage (7.2, 6.6).

10. Cage according to one of the preceding claims, **characterized in that** the grid-shaped areas or the grid body (7) have grid opening diameters of each opening of 0.5 mm to 3.2 mm, preferably having an opening diameter of each opening in the order of 0.5 mm to 0.7 mm on the outer side of the cage.

11. Cage according to one of the preceding claims, **characterized in that** the grid-shaped areas or the grid body (7) have openings or perforations with diameters of 0.5 mm to 3.2 mm in the interior of the cage.

12. Cage according to one of the preceding claims, **characterized by** a proximal connection area (6.3) for connecting the cage to an insertion instrument (8), wherein in particular the connection area (6.3) on the inner face of side walls of the cage (5) has depressions (6.3.2), which permit an angularly movable, undercutting connection to the insertion instrument (8).

13. Cage according to one of the preceding claims, **characterized in that** a connection area (6.3) in the upper face of the cage (5) has a cutout, which permits an angular mobility between cage (5) and insertion instrument (8).

14. Cage according to one of the preceding claims, **characterized in that** a proximal end face (6.3.3) has teeth (6.3.4), wherein in particular the teeth (6.3.4) are made hollow by following each other in a vertical direction on circular segments, and the teeth (6.3.4) are arranged on both sides of a proximal passage (6.3.1) formed centrally on the proximal end face.

## Revendications

1. Cage intervertébrale avec un cadre extérieur (6) composé de parties portantes (6.1, 6.2, 6.3, 6.5) massives et avec un corps de treillis intérieur, **caractérisée en ce que** le corps de treillis (7) n'est relié au cadre (6) qu'au niveau des surfaces (7.3, 7.4, 7.5, 7.6) s'étendant parallèlement dans une direction, notamment dans la direction transversale, mais n'est pas relié au cadre (6) dans les surfaces (7.8) et arêtes (7.9) s'étendant selon un angle fini par rapport à ces surfaces (7.3, 7.4, 7.5, 7.6).

2. Cage selon la revendication 1, **caractérisée en ce qu'**un cadre définissant le contour extérieur et des zones de filet ou de treillis se trouvant à l'intérieur de celui-ci sont réalisés d'un seul tenant.

3. Cage selon l'une quelconque des revendications précédentes, **caractérisée en ce qu'**elle est fabriquée par frittage, par exemple par frittage au laser.

4. Cage selon l'une quelconque des revendications 1 à 3, **caractérisée en ce qu'**elle est fabriquée par fusion de faisceau électronique, notamment par fusion sélective de faisceau électronique.

5. Cage selon l'une quelconque des revendications 1 à 4, **caractérisée en ce que** les surfaces (7.10) supérieure et inférieure du corps de treillis (7) présentent les mêmes dimensions que les espaces libres entourant les composants de cadre (6.1, 6.2, 6.3, 6.5), lesdits espaces libres entourant lesdites surfaces (7.10) du corps de treillis (7).

6. Cage selon l'une quelconque des revendications 1 à 5, **caractérisée en ce que** le cadre (6) entoure un espace creux dans lequel le corps de treillis (7) est disposé.

7. Cage selon l'une quelconque des revendications 1 à 6, **caractérisée en ce que** le cadre (4) comporte des nervures longitudinales (6.1) s'étendant dans sa direction longitudinale, les nervures longitudinales (6.1) connexes étant notamment reliées de façon centrale par les nervures transversales (6.5).

8. Cage selon l'une quelconque des revendications précédentes, **caractérisée en ce que** la zone de treillis ou le corps de treillis (6) comporte une structure en diamant.

9. Cage selon l'une quelconque des revendications précédentes, **caractérisée en ce qu'**elle comporte un passage (7.2, 6.6) traversant.

10. Cage selon l'une quelconque des revendications précédentes, **caractérisée en ce que** les zones en forme de treillis ou le corps de treillis (7) présentent un diamètre d'ouverture de treillis de chaque ouverture de 0,5 mm à 3,2 mm, comportent en l'occurrence de préférence un diamètre d'ouverture de chaque ouverture au niveau de chaque côté extérieur de la cage d'ordre de grandeur de 0,5 mm à 0,7 mm.

11. Cage selon l'une quelconque des revendications précédentes, **caractérisée en ce que** les zones en forme de treillis ou le corps de treillis (7) comportent des ouvertures ou passages traversants à l'intérieur de la cage avec des diamètres de 0,5 mm à 3,2 mm.

12. Cage selon l'une quelconque des revendications précédentes, **caractérisée par** la présence d'une zone de liaison (6.3) proximale permettant de relier la cage à un instrument d'insertion (8), notamment la zone de liaison (6.3) comportant notamment, sur le côté intérieur des parois latérales de la cage (5), des renfoncements (6.3.2) permettant d'établir une liaison détourée à angle variable avec l'instrument d'insertion (8).

13. Cage selon l'une quelconque des revendications précédentes, **caractérisée en ce qu'**une zone de liaison (6.3) comporte dans le côté supérieur de la cage (5) un détouré permettant une mobilité angulaire entre la cage (5) et l'instrument d'insertion (8).

14. Cage selon l'une quelconque des revendications précédentes, **caractérisée en ce qu'**un côté avant proximal (6.3.3) comporte des endentements (6.3.4), les endentements (6.3.4) étant notamment formés en creux successifs dans la direction verticale sur les sections circulaires et les endentements (6.3.4) étant disposés des deux côtés d'un passage (6.3.1) proximal réalisé de façon centrale au niveau du côté avant proximal.
